# DEMANDE DE BREVET EUROPEEN

(11) **EP 4 732 771 A1**
(43) Date de publication de la demande: **29.04.2026**
(21) Numéro de dépôt: 25209905.6
(22) Date de dépôt: 20.10.2025
(51) Int. Cl.: A61B 5/15, A61M 5/32, A61M 5/50

(54) **DISPOSITIF MÉDICAL COMPRENANT UNE AIGUILLE ET UN ÉTUI**

(30) Priorité: 22.10.2024 FR 2411518
(71) Demandeur: Maco Pharma, 59420 Mouvaux (FR)
(72) Inventeur: CHEBRE, Nathan, 59429 Mouvaux (FR); DEKERLE, Damien, 59420 Mouvaux (FR)
(74) Mandataire: Sayettat, Julien Christian

(57) **Abrégé**

L'invention concerne un dispositif médical (1) comprenant une aiguille (2) montée dans une embase (3) et un étui (6) présentant un logement (7) dans lequel ladite aiguille est disposée, ledit étui étant maintenu sur l'embase (3) au moyen d'une bague (8) qui est immobilisée axialement autour d'une portée proximale (9a) de ladite embase en formant entre elles une interface proximale (10a) agencée pour assurer une solidarisation en rotation de l'ensemble embase (3) / bague (8), ladite bague présentant un bord distal qui est relié à un bord proximal de l'étui (6) au moyen d'une couronne de matière (17) agencée pour pouvoir être rompue par rotation relative entre l'étui (6) et l'ensemble embase (3) / bague (8) en vue de pouvoir utiliser l'aiguille (2), l'étanchéité du logement (7) étant réalisée en combinaison au niveau de l'interface proximale (10a) et par la couronne (17) qui est continue.

## Description

L'invention concerne un dispositif médical comprenant une aiguille s'étendant entre une extrémité distale libre et une extrémité proximale montée dans une embase.

Dans une application particulière, un tel dispositif médical peut être intégré dans un système de prélèvement, qui comprend au moins une poche en communication fluidique avec l'aiguille dudit dispositif, cette dernière étant agencée pour prélever du sang à recueillir dans ladite poche.

Pour garantir la stérilité de l'aiguille avant son utilisation, ainsi que pour limiter les risques de piqûre accidentelle pour le praticien, de tels dispositifs comprennent généralement un étui qui présente un logement dans lequel ladite aiguille est disposée, en étant associé à l'embase via des moyens agencés pour se rompre lors du retrait dudit étui, et ainsi former un témoin de première ouverture dudit dispositif.

Pour ce faire, on connait des dispositifs dont l'étui est maintenu sur l'embase au moyen d'une couronne de matière formant témoin de première ouverture, en étant pour ce faire agencée pour pouvoir être rompue par rotation relative entre l'étui et l'embase, au moment où le praticien souhaite utiliser l'aiguille.

Pour faciliter sa rupture, la couronne de matière présente généralement une forme circonférentielle discontinue, en étant formée par des ponts de matière équirépartis angulairement.

Avec un tel agencement, il existe un risque de pénétration d'air extérieur dans l'étui via les espaces angulaires séparant les ponts de matière, et donc de perte de stérilité de l'aiguille disposée dans ledit étui.

Pour pallier ces inconvénients, le document JP-2017/012638 décrit un dispositif dans lequel l'embase présente une portée distale sur laquelle l'étui est associé de manière à former entre eux une interférence radiale étanche, afin d'empêcher la pénétration dans le logement de réception de l'aiguille d'air extérieur passant par la couronne frangible.

Cet agencement ne donne pas entière satisfaction, en ce qu'il requiert de former une interface d'étanchéité du côté distale de la couronne frangible, ce qui peut dégrader la facilité de retrait de l'étui du fait du serrage de ce dernier autour de l'embase.

L'invention vise à perfectionner l'art antérieur en proposant notamment un dispositif médical dans lequel l'étui est associé à l'embase de l'aiguille de manière simple en formant un témoin de première ouverture, tout en garantissant une bonne étanchéité sans venir gêner le retrait de l'étui au moment de l'utilisation.

A cet effet, selon un premier aspect, l'invention propose un dispositif médical comprenant une aiguille s'étendant entre une extrémité distale libre et une extrémité proximale montée dans une embase, ledit dispositif comprenant un étui présentant un logement dans lequel ladite aiguille est disposée, ledit étui étant maintenu sur l'embase au moyen d'une bague qui est immobilisée axialement autour d'une portée proximale de ladite embase en formant entre ladite bague et ladite portée proximale une interface proximale agencée pour assurer une solidarisation en rotation de l'ensemble embase / bague, ladite bague présentant un bord distal qui est relié à un bord proximal de l'étui au moyen d'une couronne de matière qui est agencée pour pouvoir être rompue par rotation relative entre l'étui et l'ensemble embase / bague en vue de pouvoir utiliser l'aiguille, l'étanchéité du logement de l'étui étant réalisée en combinaison au niveau de l'interface proximale et par la couronne de matière qui est continue.

Selon un second aspect, l'invention propose un système de prélèvement comprenant au moins une poche en communication fluidique avec l'aiguille d'un tel dispositif médical, ladite aiguille étant agencée pour prélever du sang à recueillir dans ladite poche.

D'autres particularités et avantages de l'invention apparaîtront dans la description qui suit, faite en référence aux figures jointes, dans lesquelles :
[Fig.1],
[Fig.2] et
[Fig.3] représentent un dispositif médical selon un mode de réalisation de l'invention, respectivement en perspective (figure 1), en vue latérale (figure 2) et en coupe axiale (figure 3), et dans lequel l'étui est associé à l'embase de l'aiguille avant utilisation ;
[Fig.3a] et
[Fig.3b] représentent le dispositif médical des figures précédentes, suivant respectivement un agrandissement de la zone A de la figure 3 (figure 3a) et le plan de coupe radiale B-B des figures 2 et 3 (figure 3b) ;
[Fig.4a] et
[Fig.4b] représentent le dispositif médical des figures précédentes durant le retrait de l'étui en vue de l'utilisation de l'aiguille, respectivement en perspective (figure 4a) et en coupe axiale (figure 4b) ;
[Fig.5] représente un système de prélèvement dans lequel un dispositif médical selon les figures précédentes est intégré.

En relation avec ces figures, on décrit ci-dessous un dispositif médical 1 comprenant une aiguille 2 s'étendant entre une extrémité distale libre 2a et une extrémité proximale 2b montée dans une embase 3, ainsi qu'un système de prélèvement intégrant un tel dispositif médical 1 et au moins une poche 4 en communication fluidique avec l'aiguille 1 dudit dispositif médical, afin de pouvoir prélever du sang à recueillir dans ladite poche.

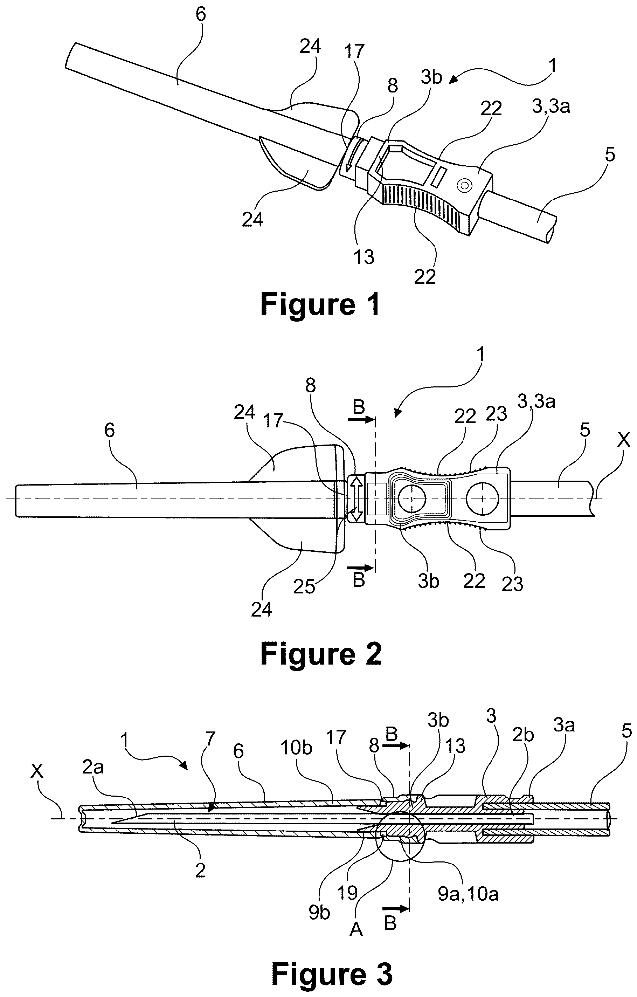

Dans la description, les termes de positionnement dans l'espace sont pris en référence au dispositif médical 1 tel que représenté sur les figures. Ainsi :
- le terme « axial » sont pris en référence à une direction parallèle à la dimension maximale de l'aiguille 2, représentée par l'axe X sur les figures 2, 3 et 4b, et le terme « radial » fait référence à une direction perpendiculaire à cet axe X ;
- les termes « proximal » et « distal » sont pris en référence au sens d'introduction de l'aiguille 2 dans une partie du corps d'un donneur, notamment d'une veine pour prélever du sang dudit donneur, et à des dispositions spatiales représentées respectivement à droite et à gauche sur les figures 2, 3 et 4b.

L'embase 3 présente une portion proximale 3a sur laquelle est montée un moyen 5 de mise en communication fluidique de l'aiguille 2 avec une poche 4 de prélèvement, notamment sous la forme d'une tubulure souple et stérilisable, ainsi qu'une portion distale 3b depuis laquelle l'extrémité libre 2a de ladite aiguille s'étend axialement.

Pour garantir la stérilité de l'aiguille 2 avant son utilisation et limiter les risques de piqûre accidentelle pour le praticien, le dispositif 1 comprend un étui 6 qui présente un logement 7 dans lequel ladite aiguille est disposée.

L'étui 6 est maintenu sur l'embase 3 au moyen d'une bague 8 qui est immobilisée axialement autour d'une portée proximale 9a formée sur la portion distale 3b de ladite embase, en formant entre ladite base et ladite portée proximale une interface proximale 10a agencée pour assurer une solidarisation en rotation de l'ensemble embase 3 / bague 8.

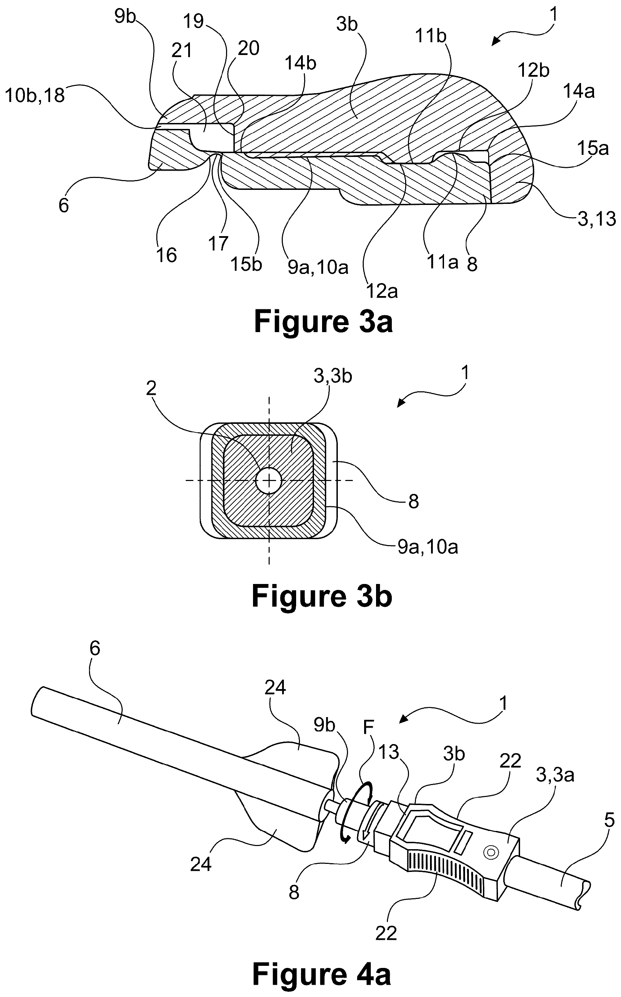

En relation avec la figure 3a, la bague 8 est immobilisée axialement autour de la portée proximale 9a au moyen d'un mécanisme formé à l'interface proximale 10a, ledit mécanisme comprenant une saillie radiale 11a, 11b qui est engagée dans une rainure 12a, 12b.

En particulier, la bague 8 comprend une rainure distale 12a qui est prolongée axialement par une saillie proximale 11a, ladite rainure distale et ladite saillie proximale étant agencées pour coopérer radialement avec respectivement une saillie distale 11b et une rainure proximale 12b formées à cet effet dans l'embase 3.

L'embase 3 présente en outre une collerette proximale 13 qui s'étend radialement autour du bord proximal 14a de la portée proximale 9a, en étant notamment formée dans le prolongement axial de la rainure proximale 12b, le bord proximal 15a de la bague 8 étant disposé en appui axial contre la collerette 13.

Par ailleurs, pour solidariser en rotation l'ensemble embase 3 / bague 8, l'interface proximale 9a entre ladite bague et ladite portée proximale présente une géométrie qui n'est pas de révolution, en étant par exemple parallélépipédique, et notamment carrée, comme représenté sur la figure 3b.

La bague 8 présente un bord distal 15b qui est relié à un bord proximal 16 de l'étui 6 au moyen d'une couronne de matière 17 formant témoin de première ouverture, et qui est agencée pour pouvoir être rompue par rotation relative entre l'étui 6 et l'ensemble embase 3 / bague 8, en vue de pouvoir utiliser l'aiguille 2.

Ainsi, avant d'utiliser le dispositif médical 1, le praticien peut en vérifier la fiabilité, et notamment son étanchéité, en contrôlant visuellement l'état de la couronne de matière 17, afin de pouvoir détruire ledit dispositif en cas de dégradation de ladite couronne de matière. Le caractère sécable permet également de confirmer que l'étui 6 n'a jamais été ouvert en ce que, si la couronne de matière 17 avait été rompue, ledit étui ne serait plus en place et l'aiguille 2 exposée.

De façon avantageuse, l'étui 6 et la bague 8 sont formés d'une seule pièce avec la couronne de matière 17, notamment par moulage d'un matériau polymère, ce qui permet d'en faciliter la fabrication.

En particulier, l'ensemble étui 6 / bague 8 / couronne 17 peut être réalisé à base de polypropylène. Selon une réalisation avantageuse, une matière particulièrement adaptée est un polypropylène homopolymère à forte rigidité possédant une déformation à la rupture inférieure à 25% et une résistance au choc Charpy inférieure à 5 kJ/m² (Référentiel Charpy « Impact Strength Notched » pour résilience au choc entaillé ISO179).

Ce matériau polymère peut également :
- contenir un colorant, afin de donner au praticien un indicatif visuel relatif à l'aiguille, par exemple son calibre, en fonction dudit colorant ; et/ou
- être transparent, afin de permettre au praticien de contrôler visuellement l'aspect de l'aiguille 2, et notamment son intégrité, au travers de l'étui 6, et ce sans avoir à ôter ledit étui.

L'étanchéité du logement 7 est réalisée en combinaison au niveau de l'interface proximale 10a et par la couronne de matière 17 qui est continue, ce qui permet d'empêcher toute pénétration d'air extérieur dans le logement via ladite couronne et en amont de ladite couronne, et ainsi de garantir la stérilité de l'aiguille 2 avant la rupture de ladite couronne et le retrait de l'étui 6.

Comme représenté notamment sur la figure 3a, l'interférence radiale du montage de la bague 8 sur la portée proximale 9a est agencée pour assurer l'étanchéité de leur interface proximale 10a, notamment par emmanchement sans jeu radial de la bague 8 autour de ladite portée proximale.

La couronne de matière 17 présente une épaisseur inférieure à celle de la bague 8 et de l'étui 6, et notamment comprise entre 30 et 100 µm, afin de faciliter sa rupture lorsque le praticien fait pivoter l'étui 6 autour de l'axe X par rapport à l'embase 3.

En relation avec les figures, l'embase 3 présente en outre une portée distale 9b qui formée sur sa portion distale 3b en prolongeant axialement la portée proximale 9a, et qui est disposée dans l'étui 6 en formant entre eux une interface distale 10b, la dimension extérieure de ladite portée distale étant inférieure à celle de ladite portée proximale.

De façon avantageuse, comme représenté sur la figure 3a, l'interface distale 10b forme un jeu radial 18 entre la portée distale 9b et l'étui 6, ce qui permet d'éviter toute résistance au niveau de ladite interface radiale lors de la rotation de l'étui 6 pour rompre la couronne 17, puis lors du retrait par translation axiale dudit étui après ladite rupture.

En particulier, la présence d'une couronne de matière 17 continue permet de s'affranchir de la réalisation d'une interférence étanche supplémentaire entre la portée radiale 9b et l'étui 6, dans la mesure où une telle couronne 17 empêche toute pénétration d'air extérieur entre l'embase 3 et ledit étui.

Pour faciliter davantage la rotation de l'étui 6, puis son retrait de l'embase 3, l'interface distale 10b susmentionnée présente une géométrie de révolution.

De même, la couronne de matière 17 est disposée autour du bord proximal 20 de la portée distale 9b en formant entre eux un jeu annulaire radial 19.

En relation avec les figures 3 et 3a, le bord proximal 20 de la portée distale 9b est relié au bord distal 14b de la portée proximale 9a par une marche radiale 21, ce qui permet de former un jeu annulaire 19 de dimensions suffisamment importantes pour faciliter la rotation et le retrait axial de l'étui 6 au moment de l'utilisation du dispositif médical 1.

L'embase 3 présente une zone de préhension manuelle qui, sur les figures, comprend deux empreintes concaves 22 formées chacune de part et d'autre de l'axe X et entre les portions proximale 3a et distale 3b de ladite embase, afin de permettre au praticien de saisir et manipuler ladite embase en pinçant deux de ses doigts sur lesdites empreintes.

En particulier, comme représenté sur la figure 2, chaque empreinte 22 comprend des nervures 23 équiréparties axialement le long de ladite empreinte, afin de limiter les glissements des doigts du praticien lorsqu'il saisit l'embase 3.

Par ailleurs, l'étui 6 est équipé d'au moins un organe 24 de préhension manuelle qui est agencé pour faciliter sa manipulation en rotation.

Sur les figures, l'organe 24 est formé au niveau du bord proximal 16 de l'étui et comprend au moins une ailette 24 qui s'étend radialement depuis la périphérie dudit étui.

De façon avantageuse, l'étui 6 est équipé de deux ailettes 24 diamétralement opposées, afin de permettre au praticien de faire tourner axialement ledit étui en disposant deux de ses doigts sur lesdites ailettes.

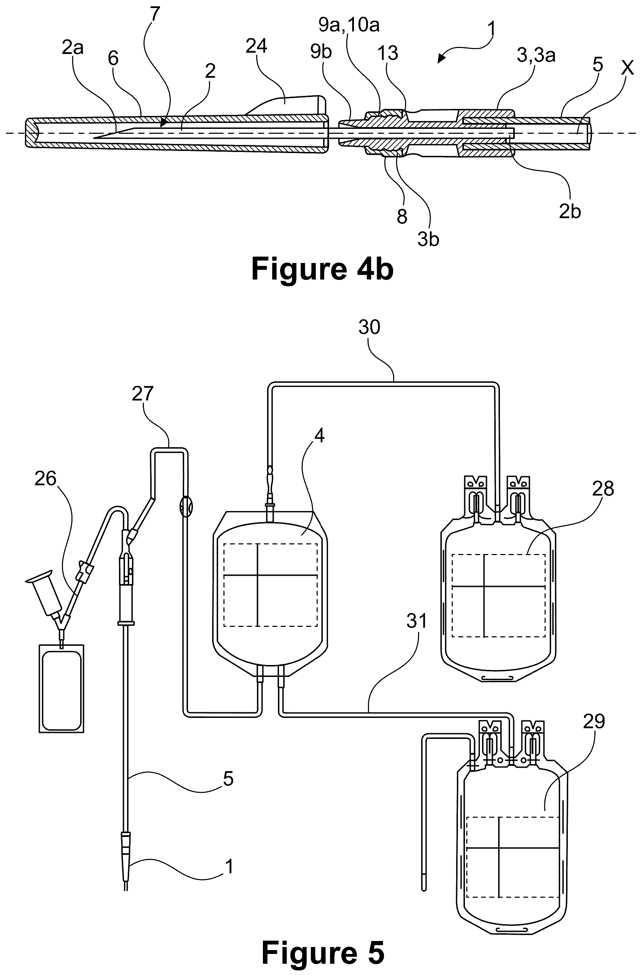

Pour ouvrir le dispositif médical 1, le praticien peut saisir l'embase 3 au moyen d'une de ses mains, en la pinçant entre deux de ses doigts au niveau de ses empreintes 22 puis, tout en maintenant ladite embase, saisir les ailettes 24 avec deux doigts de son autre main, afin de faire pivoter l'étui 6 par appui sur lesdites ailettes suivant un sens de rotation indiqué par des flèches F sur la figure 4a, jusqu'à provoquer la rupture de la couronne de matière 17.

En particulier, le dispositif médical 1 peut comprend un indicateur visuel du sens de rotation à appliquer à l'étui 6, par exemple sous forme de flèches 25 imprimées circonférentiellement sur la périphérie extérieure de la bague 8, comme représenté sur la figure 2.

On décrit à présent un système de prélèvement intégrant un tel dispositif médical 1, ledit système comprenant au moins une poche 4 en communication fluidique avec l'aiguille 2 dudit dispositif médical, ladite aiguille étant agencée pour prélever du sang à recueillir dans ladite poche.

Sur la figure 5, le système de prélèvement comprend une poche primaire 4 destinée à recueillir du sang total provenant d'un donneur, et avec laquelle l'aiguille 2 du dispositif médical 1 est mise en communication fluidique par l'intermédiaire d'une tubulure 5 souple et stérilisable.

En particulier, la tubulure 5 est raccordée à un dispositif 26 d'échantillonnage qui permet de prélever une quantité de sang du donneur pour d'autres usages médicaux, notamment des analyses sanguines complémentaires préalables à la transfusion dudit sang à un receveur, la poche primaire 4 de recueil du sang étant connectée audit dispositif d'échantillonnage par une tubulure 27 similaire à celle décrite précédemment.

Pour prélever du sang d'un donneur, le praticien introduit l'aiguille 2 du dispositif médical 1 dans une veine dudit donneur, par exemple une veine d'un bras, le sang prélevé par ladite aiguille étant ensuite acheminé vers la poche primaire 4 par l'intermédiaire des tubulures 5, 27.

Le système de prélèvement représenté comprend en outre deux poches satellites 28, 29 connectées chacune à la poche primaire au moyen de respectivement une tubulure 30, 31 souple et stérilisable, notamment similaire aux tubulures 5, 27, lesdites poches satellites étant chacune agencée pour recueillir un produit sanguin issu du sang total contenu dans ladite poche primaire.

En particulier, le système de prélèvement comprend une poche satellite supérieure 28 - respectivement inférieure 29 - destinée à recevoir le plasma - respectivement le concentré de globules rouges - du sang total recueilli dans la poche primaire 4, et connectée pour ce faire via une tubulure souple qui est associée en partie supérieure - respectivement inférieure - de ladite poche primaire.

La poche primaire 4 peut contenir un anticoagulant, tel que par exemple une solution de type CPD (Citrate Phosphate Dextrose), afin de contribuer à la conservation du sang total prélevé. De même, la poche satellite inférieure 29 peut comprendre une solution additive pour la conservation des globules rouges, par exemple de type SAGM (Saline Adénine Glucose Mannitol).

Après le prélèvement de sang sur un donneur, le système contenant le sang total prélevé sur ledit donneur, et mélangé à la solution anticoagulante dans la poche primaire 4, est placé dans un godet de centrifugation pour être centrifugé, de sorte à séparer le sang en trois couches :
- un surnageant contenant le plasma ;
- une couche intermédiaire dite « buffy coat » comprenant un mélange de plaquettes, plasma et globules rouges ; et
- un culot comprenant le concentré de globules rouges.

Par pression sur la poche primaire 4, le plasma est envoyé dans la poche satellite supérieure 28 et le concentré de globules rouges est envoyé dans la poche satellite inférieure 29, la couche « buffy coat » restant dans la poche primaire 4.

## Revendications

1. Dispositif médical (1) comprenant une aiguille (2) s'étendant entre une extrémité distale (2a) libre et une extrémité proximale (2b) montée dans une embase (3), ledit dispositif comprenant un étui (6) présentant un logement (7) dans lequel ladite aiguille est disposée, ledit étui étant maintenu sur l'embase (3) au moyen d'une bague (8) qui est immobilisée axialement autour d'une portée proximale (9a) de ladite embase en formant entre ladite bague et ladite portée proximale une interface proximale (10a) agencée pour assurer une solidarisation en rotation de l'ensemble embase (3) / bague (8), ladite bague présentant un bord distal (15b) qui est relié à un bord proximal (16) de l'étui (6) au moyen d'une couronne de matière (17) qui est agencée pour pouvoir être rompue par rotation relative entre l'étui (6) et l'ensemble embase (3) / bague (8) en vue de pouvoir utiliser l'aiguille (2), ledit dispositif médical étant **caractérisé en ce que** l'étanchéité du logement (7) de l'étui (6) est réalisée en combinaison au niveau de l'interface proximale (10a) et par la couronne de matière (17) qui est continue.

2. Dispositif médical (1) selon la revendication 1, **caractérisé en ce que** l'interférence radiale du montage de la bague (8) sur la portée proximale (9a) est agencée pour assurer l'étanchéité de l'interface proximale (10a).

3. Dispositif médical (1) selon l'une des revendications 1 ou 2, **caractérisé en ce que** l'épaisseur de la couronne de matière (17) est comprise entre 30 et 100 µm.

4. Dispositif médical (1) selon l'une quelconque des revendications 1 à 3, **caractérisé en ce que** l'interface proximale (10a) entre la bague (8) et la portée proximale (9a) présente une géométrie qui n'est pas de révolution afin de solidariser en rotation l'ensemble embase (3) / bague (8).

5. Dispositif médical (1) selon l'une quelconque des revendications 1 à 4, **caractérisé en ce que** la bague (8) est immobilisée axialement autour de la portée proximale (9a) au moyen d'un mécanisme formé à l'interface proximale (10a), ledit mécanisme comprenant une saillie radiale (11a, 11b) qui est engagée dans une rainure (12a, 12b).

6. Dispositif médical (1) selon l'une quelconque des revendications 1 à 5, **caractérisé en ce que** l'embase (3) présente une portée distale (9b) qui prolonge la portée proximale (9a), la dimension extérieure de ladite portée distale étant inférieure à la dimension extérieure de ladite portée proximale.

7. Dispositif médical (1) selon la revendication 6, **caractérisé en ce que** la couronne de matière (17) est disposée autour du bord proximal (20) de la portée distale (9b) en formant entre eux un jeu annulaire radial (19).

8. Dispositif médical (1) selon l'une des revendications 6 ou 7, **caractérisé en ce que** la portée distale (9b) est disposée dans l'étui (6) en formant entre eux un jeu radial (18).

9. Dispositif médical (1) selon l'une quelconque des revendications 1 à 8, **caractérisé en ce que** l'étui (6) est équipé d'au moins un organe (24) de préhension manuelle qui est agencé pour faciliter sa manipulation en rotation.

10. Dispositif médical (1) selon la revendication 9, **caractérisé en ce que** l'organe comprend au moins une ailette (24) s'étendant radialement depuis la périphérie de l'étui (6).

11. Dispositif médical (1) selon l'une des revendications 9 ou 10, **caractérisé en ce que** l'organe de préhension (24) est formé au niveau du bord proximal (16) de l'étui (6).

12. Dispositif médical (1) selon l'une quelconque des revendications 1 à 11, **caractérisé en ce que** l'étui (6) et la bague (8) sont formé d'une seule pièce, notamment par moulage d'un matériau polymère, notamment en polypropylène.

13. Dispositif médical (1) selon la revendication 12, **caractérisé en ce que** le polypropylène est un homopolymère possédant une déformation à la rupture inférieure à 25% et une résistance au choc Charpy inférieure à 5 kJ/m².

14. Système de prélèvement comprenant au moins une poche (4) en communication fluidique avec l'aiguille (2) d'un dispositif médical (1) selon l'une quelconque des revendications 1 à 13, ladite aiguille étant agencée pour prélever du sang à recueillir dans ladite poche.
